# EUROPEAN PATENT APPLICATION

(11) **EP 0 670 144 A1**
(43) Date of publication of application: **06.09.1995**
(21) Application number: 95102634.3
(22) Date of filing: 24.02.1995
(51) Int. Cl.: A61B 5/20, A61F 5/44, F16K 11/085

(54) **Differentiated draining valve for urinometer**

(30) Priority: 25.02.1994 IT MI940347
(71) Applicant: PRODUCTES CLINICS, S.A., E-08120 La Llagosta, Barcelona (ES); MEDINET S.p.A., I-20154 Milan (IT)
(72) Inventor: Apolet, Josef Berek, I-20146 Milan (IT)
(74) Representative: Gervasi, Gemma, Dr.

(57) **Abstract**

Device for the gathering and measuring of the catheterized patients' urine, including at least two measuring collectors bins (1,2,3) each equipped with a suitable draining duct (E₁,E₂,E₃) flowing down in only one draining valve (7) constituted by a cylindrical envelope (8) and by an inner cylindrical body (9) equipped with a hand-grip (11) at one end for the rotation manual control, said cylindrical body (9) being equipped with downflow cavities (10) on its lateral surface, said cavities being in communication, in the draining positions, with the draining ducts (E), in sequence, and being connected with the common exit duct (U) of the liquid.

## Description

The present invention concerns an improved advice for the gathering and the measuring of the catheterized patients' urine that allows the separate taking of the urine, for the analysis, in different fixed periods according to the pathological course.

The device finds a particularly interesting use in the case of patients who have had a surgical operation: in fact in such a case it is very important to control in the first hours after the operation, after the going over of the anaesthesia effect, the regular renewal of the renal function both as amount of secreted liquid and as elimination of the residual anaesthetic (or of its metabolization products) by the analysis of the urine.

For this it is necessary to have the possibility of takings differentiated in time for the analysis of the urine, in other words to drain the liquid in a way separate from the section of gathering corresponding to a fixed period.

### PRIOR ART

Collecting measuring urine devices for the catheterized patients formed by two or more bins, which allow the measure of the liquid volume excreted in a first period and in later prefixed periods , are known in the market.

In said devices each bin is made by transparent material which allows the view of the liquid inside and it is equipped with a volumetric scale which allows the immediate reading of the gathered urine amount.

A device of this kind is described in the Patents USA N° 4 265 118 and 4 291 706.

The known devices are obviously equipped with drain valves to drain the liquid after the reading of the gathered volumes during the various periods.

The drain valves of the known devices are projected in such a way to allow the only drain of all the bins at the same time.

### DESCRIPTION OF THE INVENTION

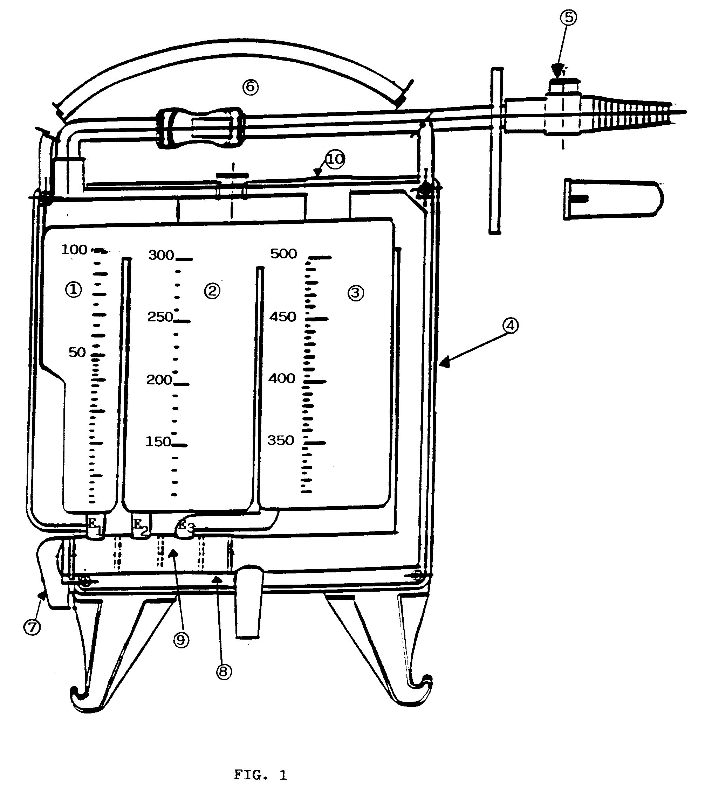

The device as a whole is shown in the fig. 1 in which the three measuring manifolds bins which subsequently overburden in the order are shown with 1, 2 and 3, the rigid assembling of the holder is shown with 4, a joint for the connection to the patient's catheter is shown with 5, a no return valve is shown with 6, an opening equipped with a filtering baffle to allow the entrance and the exit of the air avoiding the entry of dust or other pollutants inside the device is shown with 10.

The following description refers to the three bins device shown in the figures: it is clear that the device according to the invention may be made also with only two bins or with more than three.

The entrance liquid is piped into the collector 1 which communicates with a overflow connection with the successive collector 2 which is connected in turn with the 3.

The first bin which is necessary for the control of the first period following the surgical operation must give an exact measurement also for little amounts of excreted liquid and for this reason it has dimensions smaller than the successive bins. Every bin is connected on the bottom by drain ducts flowing in a drain valve 7.

This drain valve 7 is the element characterizing the present invention.

Its function is to realize a separate drain of the three bins in succession.

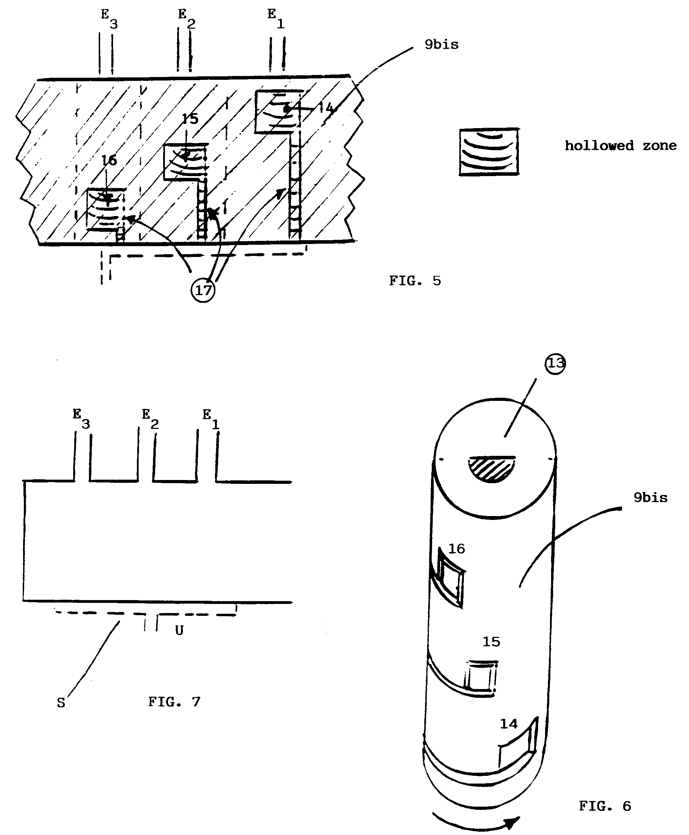

It is constituted by a cylindrical envelope 8 schematically shown in the figures 3 and 7, of rigid plastic material, put in the structure of support 4 and by a cylindrical internal body 9 put in the above mentioned cylindrical envelope and supplied with a hand-grip 11 for the draining control by rotation of the cylindrical body.

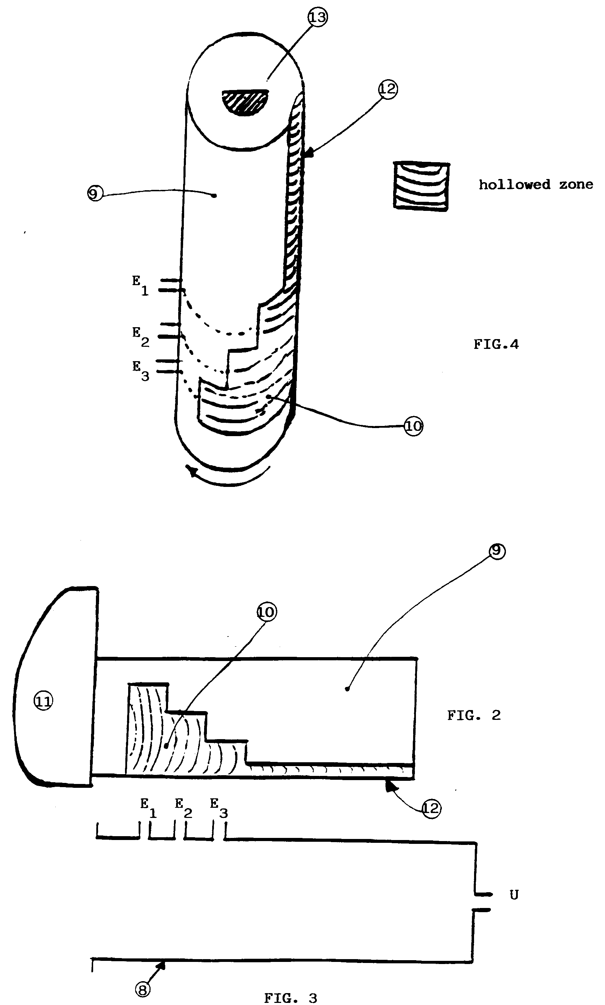

In the figures 2 and 4 a realization of the cylindrical body, with a solid structure, with superficial notches of liquid downflow, is shown respectively in longitudinal and orthogonal view.

From the above-mentioned figures it is pointed out that the cylindrical lateral surface shows a zone 10 hollowed with respect to the remaining solid surface, said zone thus constituting a not very deep depression which allows the liquid downflow, and moreover a longitudinal groove 12 debouching to the end of the cylindrical body to take the drained liquid to the exit U constituted by an axial hole situated on the bottom of the cylindrical envelope 8.

From the figures 2 and 3 it is pointed out that the cylindrical body is shorter than the cylindrical envelope and for this reason between the bottom of said envelope and the end of the cylindrical body there is an empty space.

As it is pointed out from the fig. 3, the three ducts E₁, E₂ and E₃ coming from the three measuring bins debouch in the lateral wall of the envelope 8.

When in correspondence to the above-mentioned three outlets E₁, E₂ and E₃ there is the solid surface zone of the cylindrical body the same outlets will result closed because the communication with the exit U is closed while, if in correspondence of the above-mentioned outlets there is the depression zone 10 there will be the liquid draining which is directed through the groove 12 towards the exit U.

Starting from an initial position which will be individuated to angle 0° in which all the three outlets E₁, E₂ and E₃ result closed as it is shown in the fig. 4, if the central cylindrical body is rotated in the arrow direction (that is rotating the hand-grip in the anticlockwise verse) the draining position E₁ will be reached: further rotating the hand-grip the drain E₂ and finally the drain E₃ will open.

The control of the three outlets in sequence is obtained by a rotation of the hand-grip lower than 180°.

The cylindrical body 9 is constituted by a material provided with a certain degree of elasticity for securing adherence to the cylindrical walls of the envelope 8 assuring in this way the tightness of the liquid in the solid surface zones.

The cylindrical body is equipped with an axial element 13 of rigid material which has the function to give necessary rigidity and mechanical resistance to the same body.

A further kind of the draining valve realization according to the invention provides for a cylindrical central body shown in the figures 5 and 6 respectively in longitudinal and orthogonal view.

The cylindrical body is also in this case disposed in a cylindrical cavity represented in the fig. 7 in which the three inflow ducts E₁, E₂ and E₃ coming from the three measuring bins and disposed aligned longitudinally along a generatrix of the cylinder flow.

The cylindrical cavity is equipped with a longitudinal groove S which picks up the drained liquid and directs it to the exit U.

The groove S may be in position diametrally opposite to the three inflow ducts E₁, E₂ and E₃ as it is shown in the figures 5, 6 and 7 and in nearer position too.

The valve cylindrical body is equipped on its lateral surface with three cavities 14, 15 and 16 constituted by not too deep depressions with respect to the remaining full cylindrical surface.

In the draining position these cavities are face to face with the corresponding inflow ducts E₁, E₂ and E₃.

From these cavities through the grooves 17 the liquid is directed to the common draining duct (groove S) and from here to the exit.

It is evident from the figures that rotating the cylindrical body in the direction shown by the arrow the draining will be in the order E₁, E₂ and E₃ and that only one of the three ducts E₁, E₂ and E₃ may be time by time in draining position, the other two remaining closed.

In the configuration shown in the figures 5 and 6 the three cavities are disposed, with respect to the central axis, on an arc of circle under 180°: therefore with a rotation under 180° the control cylinder may subsequently and in order assume the draining positions for E₁, E₂ and E₃.

Also in the kind of realization described above the central cylindrical body is made of a material equipped with a certain elasticity to secure the liquid tightness with respect to the cylindrical cavity and the elements of the downflow of the liquid E₁, E₂ and E₃.

Moreover it is equipped with a reinforcement axial rigid element 13.

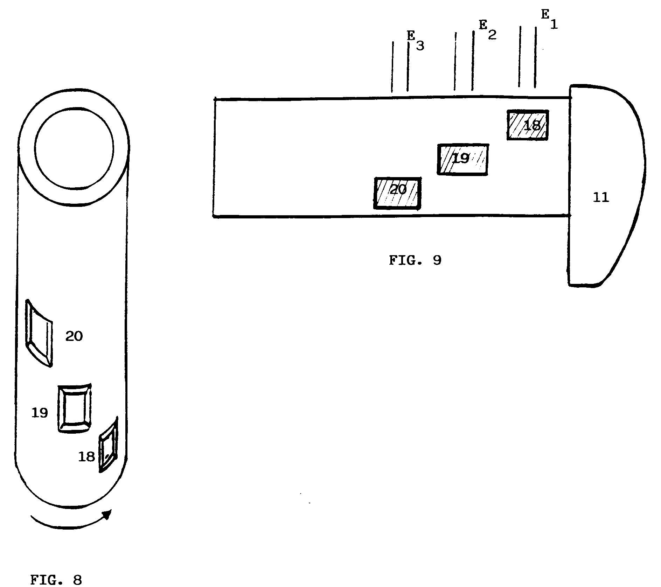

Another kind of the outlet valve realization according to the invention provides for a cylindrical central body shown in the figures 8 and 9 respectively in longitudinal and orthogonal view. This cylindrical body is hollow, with lateral walls of sufficient thickness to give the necessary rigidity and mechanical resistance to the body,it is equipped at one end with a rotation control hand-grip while the other end is open.

On the lateral surface of the hollow cylinder three openings debouching in the hollow inner zone of the cylindrical body are made. Unlike the former realizations in which the draining liquid flowed down through superficial cavities and grooves of the cylindrical body, in this case the liquid passes directly through the openings 18, 19 and 20, made in the lateral wall of the cylindrical body, in the central inner cavity and flow down from this into the space existent in the bottom of the cylindrical envelope 8 as it happens in the first described realization (see figures 2 and 3). Among the suitable materials for the manufacture of the central cylindrical body according to this last realization nylon may be named. Also the aluminium might be used as material for the hollow cylindrical body.

It is evident from the figures that rotating the cylindrical body in the direction shown by the arrow in the fig. 8, that is rotating the hand-grip 11 in clockwise verse, the draining will be in the order E₁, E₂ and E₃ and that one only of the three ducts E₁, E₂ and E₃ may be time by time in draining position, being the other two closed. Finally another realization of the valve central cylindrical body, similar to that of the figures 8 and 9, is shown in the figures 10 and 11 respectively in longitudinal and orthogonal view. The specific characteristic of this realization consists in the fact that it provides for a further control position in which all the three ducts E₁, E₂ and E₃ are open draining in this way the three bins at the same time. The hollow cylindrical body in this realization in addition to the three holes 21, 22 and 23 for the separate and following draining by the three ducts E₁, E₂ and E₃, comprises three holes 24 aligned along a generatrix of the cylinder.

These holes 24 will be obviously in correspondence of the three ducts E₁, E₂ and E₃ at the same time.

Rotating the control hand-grip in the direction shown by the arrow (that is in clockwise direction) there will be in sequence the independent draining of E₁, E₂ and E₃ while in a fourth (subsequent) position the draining happens at the same time for the three ducts E₁, E₂ and E₃.

A draining valve of this kind is advantageous because in addition to allow the draining separate and in succession of the three bins provides for a further fourth position reachable only after the three former single drains have been moved, in which there is the contemporaneous draining of the three bins. This position is not for the normal use of the apparatus which is the gathering one, measuring and taking of the urine in different temporal phases for the three bins, but for washing operations of the apparatus by running water which can flow down quickly and at the same time from all the three bins.

Moreover this draining position may be used in occasional situations that is when it is preferred to proceed to the washing of the patient's urinary bladder using in addition to the catheter already applied to the patient also the device according to the invention simply as gathering and draining container of the washing aqueous solution: in such a case it is tried obviously to accelerate the operations with the continuous and contemporaneous draining from all the three bins.

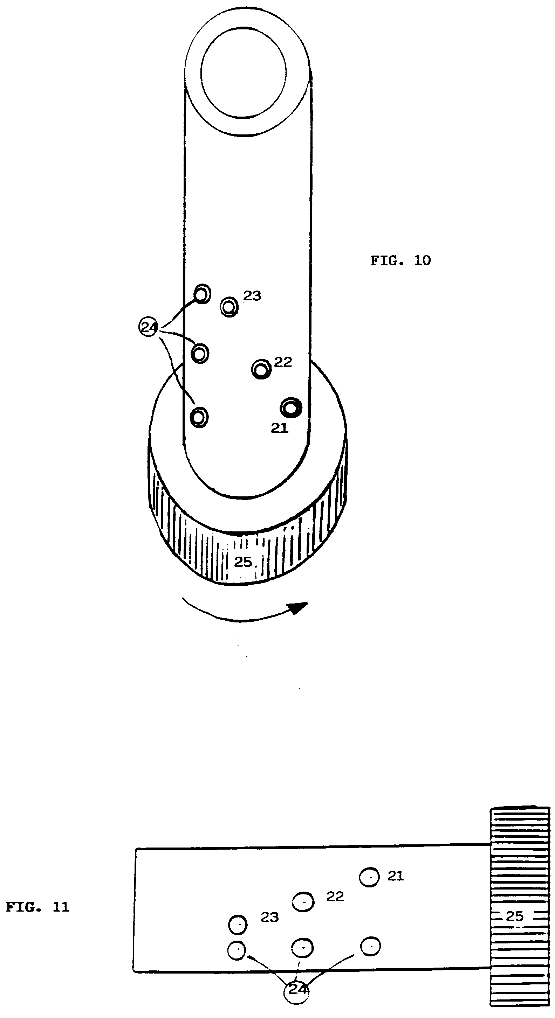

A variation of the valve central cylindrical body described above on the base of the figures 10 and 11 consists in the elimination of the draining hole 23 reducing in this way the draining positions to three.

## Claims

1. Device for the gathering and measuring of the catheterized patients' urine, including at least two measuring collectors bins, each equipped with a suitable draining duct (E) flowing down in only one draining valve (7) constituted by a cylindrical envelope (8) and by an inner cylindrical body (9) equipped with a hand-grip at one end for the rotating manual control, said cylindrical body (9) being equipped with proper openings or cavities on its lateral surface, being said openings or cavities in communication, in the draining positions, with the draining ducts (E), in sequence, and being connected with the common exit duct (U) of the liquid.

2. Device as claimed in claim 1, wherein the inner cylindrical body (9 bis) is equipped, on its lateral surface, with further draining cavities (14, 15) and (16) which are time by time faced in the draining position with corresponding draining ducts (E₁, E₂ and E₃), said draining cavities being independent one from the other, and connected by the downflow groove (17) with the liquid common exit duct (11), the ducts (E₁, E₂ and E₃) being in draining position only one by one.

3. Device as claimed in claim 1, wherein the inner cylindrical body is equipped, on its lateral surface, with openings (18, 19) and (20) communicating with the hollow inner axial part of the cylindrical body, said openings are time by time faced in the draining position with the corresponding draining ducts, (E₁, E₂ and E₃), said draining openings being independent allow the draining of the ducts (E₁, E₂ and E₃) only one by one.
